# EUROPEAN PATENT SPECIFICATION

(11) **EP 1 124 902 B1**
(45) Date of publication and mention of the grant of the patent: **02.03.2005**
(21) Application number: 99931285.3
(22) Date of filing: 11.06.1999
(51) Int. Cl.: C08L 83/12, C08G 77/46, A61K 9/58

(54) **A MEMBRANE OR MATRIX FOR CONTROLLING THE PERMEATION RATE OF DRUGS**
MEMBRAN ODER MATRIX ZUR STEUERUNG DER FREISETZUNGSGESCHWINDIGKEIT VON ARZNEISTOFFEN
MEMBRANE OU MATRICE PERMETTANT DE REGULER LA VITESSE DE PERMEATION DES MEDICAMENTS

(30) Priority: 30.06.1998 FI 981506
(43) Date of publication of application: 22.08.2001
(73) Proprietor: Schering Oy, 20210 Turku (FI)
(72) Inventor: JUKARAINEN, Harri, FIN-20610 Turku (FI); MARKKULA, Tommi, Liverpool, Merseyside L18 8 BL (GB); ALA-SORVARI, Juha, FIN-20900 Turku (FI); LEHTINEN, Matti, FIN-20760 Piispanristi (FI); RUOHONEN, Jarkko, FIN-21410 Vanhalinna (FI)
(74) Representative: Heikkilä, Hannes Antero
(86) International application number: PCT/FI1999/000511
(87) International publication number: WO 2000/000550

(56) References cited:
- EP-A1- 0 882 753
- US-A- 5 889 108
- ATN International, File CA, accession no. 126:200090, CHEMICAL ABSTRACT, Vol. 126, No. 15, 14 April 1997 (Columbus, Ohio, US), HU YUNHUA et al., "Synthesis and Drug Release Property of Polysiloxane Containing Pendant Long Alkyl Ether Group", XP002921044; & GAOFENZI XUEBAO, 1997, Vol. 1, pages 62-67
- JOURNAL OF CONTROLLED RELEASE ULMAN KATHERINE L. ET AL: 'Drug Permeability of Modified Silicone Polymers. I.Silicone-Organic Block Copolymers' vol. 10, 1989, THE NETHERLANDS, pages 251 - 260, XP002921045

## Description

The invention relates to a membrane or matrix intended for controlling the permeation rate of a drug, wherein said membrane or matrix comprises a siloxane-based elastomer composition, and to a method for the preparation of said elastomer composition.

### STATE OF THE ART

Polysiloxanes, in particular poly(dimethyl siloxane) (PDMS), are highly suitable for use as a membrane or matrix regulating the permeation rate of drugs in various drug forms, in particular in implants and IU systems. Polysiloxanes are physiologically inert, and a wide group of drugs are capable of penetrating polysiloxane membranes, which also have the required strength properties.

It is known from the literature that the adding of poly(ethylene oxide) groups, i.e. PEO groups, to a PDMS polymer may increase the permeation rate of drugs. Publication KL Ullman et al., Journal of Controlled Release 10 (1989) 251-260, describes membranes prepared from a block copolymer which contains PEO and PDMS and the penetration of various steroids through these membranes. It is noted in the publication that an increasing PEO amount in the block polymer tends to increase the penetration of hydrophilic steroids, while the penetration of lipophilic steroids decreases. The block copolymer described in the publication is very complicated in its structure and preparation, and would therefore not be facile in more extensive technical production.

### OBJECT OF THE INVENTION

The object of the invention is to provide an elastomer composition which is easy to prepare, through which a drug migrates at the desired rate, and which gives the membrane the required mechanical properties.

The object of the invention is in particular to provide an elastomer composition through which the permeation rate of drugs with hormonal action can be controlled.

### SUMMARY OF THE INVENTION

The invention thus relates to a membrane or matrix intended for controlling the permeation rate of a drug, said membrane or matrix comprising a siloxane-based elastomer composition comprising at least one elastomer and possibly a non-crosslinked polymer. The invention is characterized in that the elastomer composition comprises poly(alkylene oxide) groups and that the poly(alkylene oxide) groups are present in the elastomer or polymer as alkoxy-terminated grafts of polysiloxane units, or as blocks, the said blocks or grafts being linked to the polysiloxane units by silicon-carbon bonds, or as a mixture of these forms.

The invention also relates to a method for the preparation of a siloxane-based elastomer which comprises poly(alkylene oxide) groups and is intended for use in a membrane or matrix for controlling the permeation rate of drugs. The method is characterized in that a) a vinyl-functional polymer component and a hydride-functional component are crosslinked in the presence of a catalyst, or that b) a polymer component is crosslinked in the presence of a peroxide catalyst.

### DETAILED DESCRIPTION OF THE INVENTION

### General description of the elastomer composition

The term "elastomer composition" may stand for one single elastomer, in which case the polysiloxane units which contain poly(alkylene oxide) groups are present in the said elastomer.

According to another embodiment, the elastomer composition may be made up of two elastomers which are interlaced, one inside the other. In this case the first elastomer comprises poly(alkylene oxide) groups so that the poly(alkylene oxide) groups are present in the said elastomer either as alkoxy-terminated grafts of polysiloxane units or as blocks, the said grafts or blocks being linked to the polysiloxane units by silicon-carbon bonds. The poly(alkylene oxides) may also be present as a blend of the options mentioned. The second elastomer may be a siloxane-based elastomer, suitably a poly(dimethylsiloxane)-based elastomer. The said second elastomer may possibly also comprise poly(alkylene oxide) groups. These poly(alkylene oxide) groups may also be present either as alkoxy-terminated grafts of poly(dimethyl siloxane) units or as blocks, the said grafts or blocks being linked to the poly(dimethyl siloxane) units by silicon-carbon bonds. The poly(alkylene oxides) may also in this elastomer be present as a blend of the options mentioned above.

According to a third embodiment, the elastomer composition may be a blend which comprises a siloxane-based elastomer, which is, for example, made up of PDMS, and at least one straight-chain polysiloxane copolymer which comprises poly(alkylene oxide) groups. In this case the poly(alkylene oxide) groups are present in the said polymer either as alkoxy-terminated grafts of polysiloxane units or as blocks, the said grafts or blocks being linked to the polysiloxane units by silicon-carbon bonds. The poly(alkylene oxide) groups may, of course, also be present in the polymer as a blend of the forms mentioned. In this embodiment, also the siloxane-based elastomer may comprise poly(alkylene oxide) groups, in which case these poly(alkylene oxide) groups are present in the elastomer either as alkoxy-terminated grafts of polysiloxane units or as blocks, the said blocks or grafts being linked to the polysiloxane units by silicon-carbon bonds. The poly(alkylene oxide) groups may also be present as a blend of the forms mentioned.

Of course, the elastomer composition may also be made up of two elastomers interlaced one inside the other, as above, and at least one straight-chain polysiloxane copolymer which comprises poly(alkylene oxide) groups.

The poly(alkylene oxide) groups of the elastomer composition may suitably be, for example, poly(ethylene oxide) groups (PEO groups).

The polysiloxane units of the elastomer composition are preferably groups having the formula

-(SiR'R''O)_{q}SiR'R''-

where R' and R'' are
- partly free groups, which are the same or different and which are a lower alkyl group, or a phenyl group, in which case the said alkyl or phenyl groups may be substituted or unsubstituted, or alkoxy-terminated poly(alkylene oxide) groups having the formula 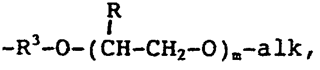 where alk is a lower alkyl group, suitably methyl, R is hydrogen or a lower alkyl, m is 1...30, and R³ is a straight or branched C₂ - C₆ alkyl group,
- partly bonds, formed from the hydrogen or alkylene groups, to other polymer chains in the elastomer, and
- possibly partly unreacted groups, such as hydrogen, vinyl or vinyl-terminated alkene, and
- q is 1...3000.

The term "lower alkyl" stands here and generally in the description of the present invention for C₁ - C₆ alkyl groups.

The above-mentioned free R' and R'' groups are suitably a lower alkyl group, preferably methyl.

The term "poly(alkylene oxide) group" means that said group comprises at least two alkyl ether groups successively connected to each other.

According to a preferred embodiment, the poly(alkylene oxide) groups are present in the elastomer in the form of poly(alkylene oxide) blocks having the formula 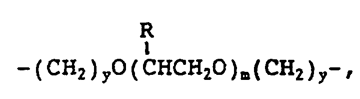 or 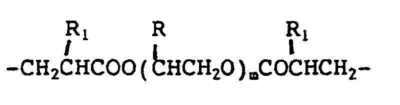 where R is hydrogen, a lower alkyl or a phenyl,
R₁ is hydrogen or a lower alkyl, y is 2...6, and m is 1...30.

The elastomer composition suitably contains a filler, such as silica, in order that the membrane should obtain a sufficient strength.

The word "membrane" means the same as film.

General description of the method for the preparation of the elastomer composition

According to a preferred embodiment, the novel elastomer is prepared by crosslinking, in the presence of a catalyst, a vinyl-functional polymer component and a hydride-functional siloxane component.

By crosslinking is meant the addition reaction of the hydride-functional siloxane component with the carbon-carbon double bond of the vinyl-functional polymer component.

According to another embodiment, the elastomer is prepared by crosslinking the polymer in the presence of a peroxide catalyst. In this case the vinyl and methyl groups react with each other and form carbon-carbon bonds. A crosslink may also be formed between two methyl groups or between two vinyl groups.

For crosslinking, the amounts of the components are preferably selected so that the ratio of the molar amounts of the hydrides and the double bonds is at least 1.

The vinyl-functional polymer component is
a) a vinyl-functional polysiloxane having the formula

   R'-SiR'R''O(SiR'R''O)ᵣSiR'R''R'

   where R' and R'' are the same or different, and are a lower alkyl group, or a phenyl group, in which case the said alkyl or phenyl group may be substituted or unsubstituted, and where some of the substituents R' and/or R'' have been substituted for by vinyl groups, and r is 1...27000, or
b) an alkenyl terminated polysiloxane-based block copolymer having the formula

   T(AB)ₓAT (I),

   where
   A = -(SiR'R''O)_{q}SiR'R''-, where R' and R'' are the same or different and are a lower alkyl group, or a phenyl, in which case the said alkyl or phenyl group may be substituted or unsubstituted;
   B is a poly(alkylene oxide) having the formula 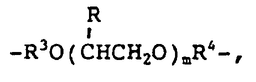 or 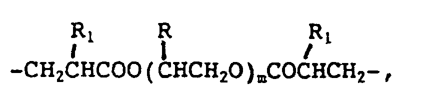 and T is 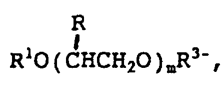 or 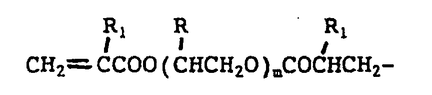 where
   R is hydrogen, a lower alkyl or phenyl, R₁ is hydrogen or a lower alkyl, R³ and R⁴ are the same or different and are straight-chain or branched C₂ - C₆ alkylene groups,
   R¹ is a straight-chain or branched C₂ - C₆ alkenyl group, m is 1...30, q is 1...3000, and x is 0...100, or
c) a vinyl-functional polysiloxane copolymer having the formula

   R'-SiR'R''O(SiR'R''O)ᵣ(SiR'R''O)ₚSiR'R''-R'

   - where in the first block R' and R" are the same or different and are a lower alkyl group, or a phenyl group, in which case the said alkyl or phenyl group may be substituted or unsubstituted, and where some of the substituents R' and/or R'' have been substituted for by vinyl groups, and r is 1...27000, and
   - where in the second block R' is a lower alkyl group, or an alkoxy-terminated poly(alkylene oxide) group having the formula 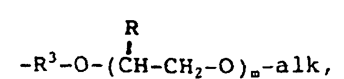 where alk is a lower alkyl group, suitably methyl, R is hydrogen or a lower alkyl group, R³ is a straight or branched C₂ - C₆ alkyl, and m is 1...30, or R' is a phenyl group, in which case the said alkyl or phenyl group may be substituted or unsubstituted, and R'' is a lower alkyl or a phenyl group, in which case the said alkyl or phenyl group may be substituted or unsubstituted, and p is 1...5000, or
d) α,ω-dialkenyl poly(alkylene oxide) having the formula 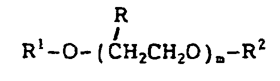 where R¹ and R² are the same or different straight-chain or branched C₂ - C₆ alkenyl groups, R is hydrogen or a lower alkyl, and m is 1...30, or
e) a blend of at least two of the above-mentioned components a) - d).

If the formula of the vinyl-functional polysiloxane copolymer is, in accordance with the above description, R'-SiR'R''O(SiR'R''O)ᵣ(SiR'R''O)ₚSiR'R''-R', it should be noted that the formula is a kind of gross formula, in which the blocks in successive parentheses may appear in any order in relation to one another. Furthermore, it is preferable that both a vinyl group and the above-mentioned alkoxy-terminated poly(alkylene oxide) group are not bonded to one and the same Si atom.

The hydride-functional component is
a) a hydride-functional siloxane, which may be straight-chain, star shaped, branched or cyclic, or
b) a hydride-terminated siloxane-based block copolymer having the formula

   T(BA)ₓBT (II),

   where
   T = H-SiR'R''O(SiR'R''O)_{q}SiR'R''-,
   A = -SiR'R''O(SiR'R''O)_{q}SiR'R''-, where R' and R" are the same or different and are a lower alkyl group or a phenyl group, in which case the said alkyl or phenyl group may be substituted or unsubstituted;
   B is a poly(alkylene oxide) having the formula 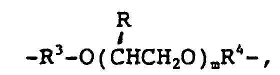 or 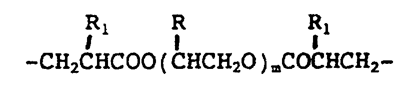 where R is hydrogen, a lower alkyl or a phenyl, R₁ is hydrogen or a lower alkyl, R³ and R⁴ are the same or different and are straight-chain or branched C₂ - C₆ alkyl groups, m is 1...30, q is 1...3000, and x is 0...100, or
c) a blend of the above-mentioned components a) and b).

According to one embodiment, the hydride-functional siloxane copolymer may be straight-chain, in which case its formula is

R'-SiR'R''O(SiR'R''O)ᵣSiR'R''R'

where R' and R" are the same or different and are a lower alkyl group, or a phenyl group, in which case the said alkyl or phenyl group may be substituted or unsubstituted, and where some of the substituents R' and/or R'' have been substituted for by hydrogen, and r is 1...27000.

The vinyl-functional polymer component may contain a filler, suitably silica.

The catalyst to be used in the crosslinking is suitably a noble metal catalyst, most commonly a platinum complex in alcohol, xylene, divinyl siloxane or cyclic vinyl siloxane. An especially suitable catalyst is a Pt(0)-divinyl-tetramethyl disiloxane complex.

The elastomer composition made up of two elastomers is prepared so that initially a first elastomer is formed, whereafter a second elastomer is formed by crosslinking in the presence of the first elastomer. Thus the second elastomer will penetrate through the first elastomer.

The elastomer composition which comprises an elastomer and a straight-chain polymer is prepared, for example, by blending a vinyl-functional polymer component, a hydride-functional component, and a polymer which has no vinyl or hydride groups. In the crosslinking, the vinyl-functional polymer component and the hydride-functional component form an elastomer, but the polymer component which does not contain the said functional groups will not take part in the crosslinking reaction but will remain, in a straight-chain form, inside the elastomer.

### EXPERIMENTAL SECTION

The invention is described below in greater detail with the help of examples.

Elastomer compositions of different types (A - J) were prepared. Of most composition types there were prepared different compositions which differed one from another with respect to the PEO amount. Elastomer membranes representing the different compositions were tested with respect to the permeation rates of various drugs.

### Elastomer compositions prepared

In the elastomer compositions A - H described below, an addition reaction between vinyl groups and silyl hydride groups was used for the crosslinking, i.e. for producing a network structure. The hydride-functional siloxane polymer serving as the crosslinking agent contained at least two Si-H groups, which reacted with the carbon-carbon double bond of the polymer to be crosslinked. Membranes made from elastomer compositions I and J were prepared by using peroxide as the catalyst for crosslinking, in which case the vinyl or methyl groups reacted, forming carbon-carbon bonds. In all the composition types except composition types A, D, F and H, there was first prepared a basic polymer blend, in which case all of the vinyl-containing polymers and the fillers, or vinyl-containing polymers which contained a filler, were mixed together. The filler used was silica. Composition types A, D, F and H had only one vinyl-containing polymer each, and thus they themselves were basic polymers. The basic polymer blend was divided into portions I and II. The catalyst was added to portion I and the crosslinking agent and the inhibitor to portion II. Portions I and II were combined immediately before the crosslinking. The obtained blend was crosslinked at a temperature which was higher than the decomposition temperature of the inhibitor and at which the crosslinking reaction took place at the desired velocity.

A blend can be made of the compositions also directly in one step, in which case the ingredients can be added in the following order: vinyl-containing polymers, inhibitor, catalyst and crosslinking agent.

The following table describes elastomer membranes of different composition types and their initial components.

**Table 1**

| **Composition type** | **Polymers containing vinyl groups in the basic polymer blend** | **Crosslinking agent** |
|---|---|---|
| A | α,ω-divinyl ether poly(ethylene oxide)-poly(dimethyl siloxane) multi-block copolymer (PEO-(-PDMS-PEO)ₙ) | Hydride-functional siloxane |
| | | |
| B | PEO-(PDMS-PEO)ₙ and a siloxane polymer containing a filler | Hydride-functional siloxane |
| | | |
| C | FEO-(PDMS-PEO)ₙ together or separately with a siloxane polymer which does or does not contain a filler | α,ω-bis(dimethyl silyl hydride)-poly(dimethyl siloxane)-poly(ethylene oxide) multiblock copolymer (PDMS-(PEO-PDMS)ₙ) together or separately with a hydride-functional siloxane. |
| | | |
| D | α,ω-divinyl ether poly(ethylene oxide (PEODIVI) | Hydride-functional siloxane |
| | | |
| E | PEODIVI and a siloxane polymer which does or does not contain a filler | Hydride-functional siloxane |
| | | |
| F | PEO-grafted dimethyl siloxane-methyl vinyl siloxane copolymer (PDMS-PEO graft copolymer) | Hydride-functional siloxane |
| | | |
| G | PDMS-PEO graft copolymer and a siloxane polymer which does or does not contain a filler | Hydride-functional siloxane |
| | | |
| H | α,ω-diallyl ether poly(ethylene oxide)-poly(dimethyl siloxane) multi-block copolymer (APEO-(-PDMS-APEO)ₙ) | Hydride-functional siloxane |
| | | |
| I | PEO-(PDMS-PEO)ₙ and a siloxane polymer which does or does not contain a filler | Peroxide |
| | | |
| J | PDMS-PEO graft copolymer together or separately with a siloxane polymer which does or does not contain a filler | Peroxide |

### EXAMPLE 1

### Elastomer membrane prepared from composition type A

Ingredients used for the preparation of the elastomer membrane:
- α,ω-divinyl ether PEO-PDMS block copolymer where the amount of PEO was 27.0 % by weight and the vinyl content was 0.186 mmol/g.
- Platinum catalyst Silopren U Katalysatoren Pt-D (Bayer AG), which had a platinum-siloxane complex in a vinyl-containing siloxane matrix. The platinum content was 1 % by weight and the vinyl content was 0.5 mmol/g.
- Crosslinking agent α,ω-di(trimethyl silyl) dimethyl siloxane-hydromethyl siloxane (DMS-HMS) copolymer Silopren U Vernetzer 730 (Bayer AG) having a Si-H content of 7.1 mmol/g, a molar mass of 2800 g/mol and a DMS group to HMS group ratio of 1:1.
- Inhibitor 1-ethinyl-1-cyclohexanol (ETCH, Aldrich) having a decomposition temperature of +40 °C.

The PEO(-PDMS-PEO)ₙ which was used as the initial substance was prepared as follows:

50 g of anhydrous α,ω-divinyl ether poly(ethylene oxide) (PEODIVI) having a molar mass of 268 g/mol was weighed into a three-necked flask. In addition, 129.87 g of α,ω-bis(dimethyl silyl hydride) poly(dimethyl siloxane) (PDMSDIH, Mₙ = 717 g/mol) and 30 % by weight of toluene dried by distillation were weighed into the same vessel. Since vinyl groups were present in excess (3 %) in the reaction, in the final product vinyl groups were obtained at both ends, which was essential for the subsequent crosslinking. The reaction solution was stirred over a magnetic stirring plate at 200 rpm, and dry oxygen was directed through the solution in order to prevent the deactivation of the catalyst. The reaction solution was heated to 50 °C, whereafter the catalyst (Pt(0) divinyl-tetramethyl disiloxane complex) was added to the solution through the septum. The amount of platinum was 30 ppm, calculated from the amount of reactants. Thereafter the polymerization was monitored by means of IR until the reactions were complete (loss of the Si-H peak at 2130 cm¹), which took approximately 4 h. After the polymerization, the toluene was distilled off from the solution by raising the temperature to 65 °C and by lowering the pressure to 5 mbar for a period of 1 h.

In the preparation of the elastomer, two blends were first prepared, portions I and II. Portion I contained PEO-(PDMS-PEO)ₙ and the platinum catalyst. Portion II contained PEO-(PDMS-PEO)ₙ, the crosslinking agent and the inhibitor. Portions I and II were combined by mixing immediately before the crosslinking.

The amounts of the ingredients in the composition example in the final blend to be crosslinked were as follows:
- Basic polymer PEO-(PDMS-PEO)ₙ 94.87 % by weight
- Platinum catalyst 0.1 % by weight
- Crosslinking agent 5.00 % by weight
- Inhibitor 0.03 % by weight

Portion I was prepared using a chamber mixer. 5.489 g of the basic polymer and 0.011 g of the platinum catalyst were weighed into the mixing chamber. The ingredients were agitated until the blend was homogeneous.

The crosslinking agent and the inhibitor were combined before being mixed with portion II. The mixture of the crosslinking agent and the inhibitor was prepared by weighing 0.059 g of ETCH and 9.941 g of Silopren U Vernetzer 730 into a glass vessel and by stirring the mixture in a water bath of +37 °C until ETCH had dissolved completely in the crosslinking agent. The amount of inhibitor in the mixture was 0.59 % by weight.

Portion II was prepared using a chamber mixer. The mantle of the chamber mixer was cooled by water circulation to a point below room temperature, whereupon the temperature increase due to friction did not raise the temperature to the decomposition temperature of the inhibitor. 4.947 g of PEO-PDMS block copolymer and 0.553 g of the mixture of the crosslinking agent and the inhibitor were weighed into the mixing chamber. The ingredients were agitated until the blend was homogeneous.

Portions I and II were combined immediately before the crosslinking, by adding 5 grams of portion I and 5 grams of portion II into the mixing chamber of the chamber mixer. The ingredients were agitated until the blend was homogeneous. The blend was recovered and was drawn into vacuum to remove air bubbles. Four batches of 2 g of the blend were weighed and crosslinked successively in a hot-press.

The weighed blend was placed between two FEP release membranes in the center of a round metal form having a thickness of 0.4 mm and an inner diameter of 8 cm. The blend, together with the forms and the FEP membranes, was placed between the compression surfaces of the hot-press, which surfaces had been heated in advance to +115 °C. The surfaces were pressed together and were kept pressed at a pressure of 200 bar for 5 minutes. The pressure was released and the membrane was allowed to set at room temperature for 24 hours. Round test pieces having a diameter of 22 mm were cut out from the membranes by means of a puncher.

### EXAMPLE 2

### Elastomer membrane prepared from composition type B

Ingredients used for the preparation of the elastomer membrane:
- The PEO(-PDMS-PEO)ₙ was the same as in Example 1, except that the amount of PEO had been increased to 28.0 % by weight and the vinyl content to 0.24 mmol/g by increasing the proportion of PEODIVI in the synthesis of the block copolymer.
- The catalyst, the crosslinking agent and the inhibitor were the same as in Example 1.

The siloxane polymer which contained filler was a dimethyl siloxane-vinyl methyl siloxane (DMS-VMS) copolymer containing a silica filler and having a molar mass of Mₙ = 400,000 g/mol. The vinyl content of the blend was 0.011 mmol/g. There was 36 % by weight of silica mixed in the polymer, and the silica was surface-treated with α,ω-bis(dimethyl hydroxysilyl) poly(dimethyl siloxane) (M = 520 g/mol), which was present in an amount of 12 % by weight in the blend.

The amounts of ingredients in the composition example were as follows:
- PEO(-PDMS-PEO)ₙ 32.8 % by weight
- DMS-VMS copolymer containing a silica filler, 60.9 % by weight
- Platinum catalyst 0.1 % by weight
- Crosslinking agent 6.19 % by weight
- Inhibitor 0.03 % by weight

First the basic polymer blend was prepared in a chamber mixer. 4.2 grams of the PEO(-PDMS-PEO)ₙ block copolymer and 7.8 grams of the DMS-VMS copolymer containing a silica filler were weighed into the mixing chamber. The ingredients were agitated until the blend was homogeneous.

Portion I was prepared as in Example 1.

The combining of the crosslinking agent and the inhibitor was done, as in Example 1, before mixing with portion II, except that ETCH was weighed in an amount of 0.048 g and Silopren U Vernetzer 730 in an amount of 9.952 g. The amount of inhibitor in the blend was 0.48 % by weight.

Portion II was prepared as in Example 1, except that the basic polymer blend was weighed in an amount of 4.816 grams and the mixture of the crosslinking agent and the inhibitor in an amount of 0.684 grams.

Portions I and II were combined as in Example 1. Four batches of 2.1 g of the blend were weighed and were crosslinked successively in a hot-press, as in Example 1.

### EXAMPLE 3

### Elastomer membrane prepared from composition type C

Ingredients used for the preparation of the elastomer membrane:
- The PEO(-PDMS-PEO)ₙ was the same as in Example 2. The catalyst and the inhibitor were the same as in Examples 1 and 2.
- The dimethyl siloxane-vinyl methyl siloxane (DMS-VMS) copolymer containing a silica filler was the same as in Example 2.
- The crosslinking agent used was a PDMS-(-PEO-PDMS)ₙ copolymer having a Si-H content of 0.26 mmol/g, and the amount of PEO in it was 23.6 % by weight.

The said crosslinking agent was prepared as follows:

40 g of an anhydrous α,ω-divinyl ether poly(ethylene oxide) (PEODIVI) having a molar mass of 246.3 g/mol was weighed into a three-necked flask. In addition, 129.4 g of α,ω-bis(dimethyl silyl hydride) poly(dimethyl siloxane) (PDMSDIH, Mₙ = 717 g/mol) and 30 % by weight of toluene dried by distillation were weighed into the same vessel. Since dimethyl silyl hydride groups were present in excess (10 %) in the reaction, dimethyl silyl hydride groups were obtained at both ends in the final product. The reaction solution was stirred over a magnetic stirring plate at 200 rpm, and dry oxygen was directed through the solution to prevent the deactivation of the catalyst. The reaction solution was heated to 50 °C, whereafter the catalyst (Pt(0) divinyl-tetramethyl siloxane complex) was added to the solution through the septum. The amount of platinum was 30 ppm, calculated from the amount of the reactants. Thereafter the polymerization was monitored by means of IR until the reactions were complete (loss of the vinyl peak at 1600 cm¹), which took approximately 4 h. After the polymerization, the toluene was removed from the solution by distillation by raising the temperature to 65 °C and by lowering the pressure to 5 mbar for a period of 1 h.

The amounts of the ingredients in the composition example were as follows:
- PEO(-PDMS-PEO)ₙ 1.10 % by weight
- DMS-VMS containing a silica filler, 85.50 % by weight
- Platinum catalyst 0.10 % by weight
- Crosslinking agent α,ω-bis-(dimethyl silyl hydride) PEO-PDMS 13.27 % by weight
- Inhibitor 0.03 % by weight

First the basic polymer blend was prepared in a chamber mixer. 0.15 grams of the α,ω-divinyl ether PEO-PDMS block copolymer and 11.85 grams of the DMS-VMS copolymer containing a silica filler were weighed into the mixing chamber. The ingredients were agitated until the blend was homogeneous.

Portion I was prepared as in Example 1. The combining of the crosslinking agent and the inhibitor was done, as in Example 1, before mixing with portion II, except that ETCH was weighed in an amount of 0.022 g and PDMS-(PEO-PDMS)ₙ. block copolymer in an amount of 9.978 g instead of Vernetzer 730. The amount of inhibitor in the blend was 0.22 % by weight.

Portion II was prepared as in Example 1, except that the basic polymer blend was weighed in an amount of 4.04 grams and the mixture of the crosslinking agent and the inhibitor in an amount of 1.46 grams.

Portions I and II were combined as in Example 1. Four batches of 2.1 g of the blend were weighed and were successively crosslinked in a hot-press, as in Example 1.

### EXAMPLE 4

### Elastomer membrane prepared from composition type D

Ingredients used for the preparation of the elastomer membrane:
- α,ω-divinyl ether poly(ethylene oxide) (PEODIVI) (polyethylene glycol divinyl ether, Aldrich, Mₙ = 240 g/mol). The vinyl amount obtained by titration was 7.4 mmol/g.
- Catalyst Gelest SIP 6831.0, platinum-siloxane complex in xylene, platinum content 2.25 % by weight.
- The crosslinking agent and the inhibitor were the same as in Example 1.

The amounts of the ingredients in the composition example were as follows:
- PEODIVI 52.231 % by weight
- Platinum catalyst 0.045 % by weight
- Crosslinking agent 47.694 % by weight
- Inhibitor 0.030 % by weight

First a mixture of the crosslinking agent and the inhibitor was prepared as in Example 1, except that the inhibitor was weighed in an amount of 0.0063 grams and the crosslinking agent in an amount of 9.9937 grams. The amount of inhibitor in the mixture was 0.063 % by weight.

5.2231 grams of PEODIVI and 0.0045 grams of the platinum catalyst were mixed together in a glass vessel. 4.772 grams of the mixture of the crosslinking agent and the inhibitor was mixed into it.

Eight batches of 0.8 g of the blend were weighed into flat-bottomed aluminum forms having a diameter of 5 cm and having a FEP membrane on the bottom. The forms were placed under a 100 mbar vacuum at +115 °C for a period of 15 minutes. Test pieces were cut out from the elastomer obtained.

### EXAMPLE 5

### Elastomer membrane prepared from composition type E

Ingredients used for the preparation of the elastomer membrane:
- PEODIVI, the same as in Example 4.
- DMS-VMS copolymer, the same as in Example 2.

The catalyst, the crosslinking agent and the inhibitor were the same as in Example 1.

The amounts of the ingredients in the composition example were as follows:
- PEODIVI 11.37 % by weight
- DMS-VMS copolymer 64.46 % by weight
- Platinum catalyst 0.1 % by weight
- Crosslinking agent 24.03 % by weight
- Inhibitor 0.03 % by weight

First, a mixture of the crosslinking agent and the inhibitor was prepared, as in Example 1, except that the inhibitor was weighed in an amount of 0.0125 grams and the crosslinking agent in an amount of 9.9875 grams. The amount of inhibitor in the mixture was 0.125 % by weight.

1.138 grams of PEODIVI and 6.446 grams of DMS-VMS copolymer were mixed together in a chamber mixer. 0.01 grams of platinum catalyst was added, and the blend was agitated until homogeneous. 2.406 grams of the mixture of the crosslinking agent and the inhibitor was added and the blend was agitated until homogeneous.

Four batches of 2.1 g of the blend were weighed and were successively crosslinked in a hot-press, as in Example 1.

### EXAMPLE 6

### Elastomer membrane prepared from composition type F

Ingredients used for the preparation of the elastomer membrane:
- PDMS-PEO graft copolymer having a vinyl concentration of 0.0743 mmol/g and a PEO content of 1.28 % by weight.
- The catalyst, the crosslinking agent and the inhibitor were the same as in composition A.

The PDMS-PEO graft copolymer used was prepared as follows:

600 g of octamethyl cyclotetrasiloxane (D₄), 9.28 g of poly(dimethyl siloxane)-poly(ethylene oxide) graft copolymer (Gelest, DBE-821, containing 80 % by weight PEO), 6.18 g of dimethyl vinyl silyl end-blocked PDMS (end-blocker, Bayer Silopren U2), and 3.1 g of tetramethyl tetravinyl cyclotetrasiloxane were weighed. The reactor was nitrogenated, the weighed chemicals were poured in, and stirring was started. The inside temperature of the reactor was raised to 135 °C, and the catalyst (potassium siloxanolate, 0.9 ml, 20 ppm K⁺) was added to the reaction solution. The viscosity of the reaction solution began to increase vigorously, and at 1 h from the adding of the catalyst it was possible to deactivate the catalyst by increasing the reactor pressure to 2 bar for a period of 15 minutes by means of carbon dioxide. Thereafter the light cyclic compounds (13 % by weight) were removed from the reaction solution by distillation (10 mbar, 30 min, 135 °C). Product Mₙ = 190,000 g/mol.

The amounts of the ingredients in the composition example were as follows:
- Basic polymer PDMS-PEO graft copolymer 96.10 % by weight
- Platinum catalyst 0.5 % by weight
- Crosslinking agent 3.06 % by weight
- Inhibitor 0.34 % by weight

The combining of the crosslinking agent and the inhibitor was done as in Example 1, except that ETCH was weighed in an amount of 1.0 g and Silopren U Vernetzer 730 in an amount of 9.0 g. The amount of inhibitor in the mixture was 10 % by weight.

9.61 grams of the PDMS-PEO graft copolymer and 0.05 grams of the platinum catalyst were mixed together. 0.34 grams of the mixture of the crosslinking agent and the inhibitor was added and the blend was stirred until homogeneous.

Four batches of 2.1 g of the blend were weighed and were successively crosslinked in a hot-press, as in Example 1.

### EXAMPLE 7

### Elastomer membrane prepared from composition type G

Ingredients used for the preparation of the elastomer membrane:
- The PDMS-PEO graft copolymer was the same as in Example 6.
- The DMS-VMS copolymer was the same as in Example 2.
- The catalyst, the crosslinking agent and the inhibitor were the same as in Example 1.

The amounts of the ingredients in the composition example were as follows:
- PDMS-PEO graft copolymer 26.75 % by weight
- DMS-VMS copolymer 72.31 % by weight
- Platinum catalyst 0.10 % by weight
- Crosslinking agent 0.81 % by weight
- Inhibitor 0.03 % by weight

The combining of the crosslinking agent and the inhibitor was done as in Example 1, except that ETCH was weighed in an amount of 0.36 g and Silopren U Vernetzer 730 in an amount of 9.64 g. The amount of inhibitor in the mixture was 3.6 % by weight.

2.675 grams of the PDMS-PEO graft copolymer and 7.231 grams of the DMS-VMS copolymer containing a filler were mixed together. 0.01 grams of the platinum catalyst was added and the blend was stirred until homogeneous. 0.084 grams of the mixture of the crosslinking agent and the inhibitor was added and the blend was stirred until homogeneous.

Four batches of 2.1 g of the blend were weighed and were successively crosslinked in a hot-press, as in Example 1.

### EXAMPLE 8

### Elastomer membrane prepared from composition type H

Ingredients used for the preparation of the elastomer membrane:
- APEO-(-PDMS-APEO)ₙ, where the amount of PEO was 10.3 % by weight and the vinyl content 0.063 mmol/g.
- The catalyst was the same as in Example 4.
- The inhibitor was the same as in Example 1.
- The crosslinking agent was a DMS-HMS copolymer which contained 22.5 % by weight methyl hydride siloxane groups (Gelest).

The APEO-(-PDMS-APEO)ₙ used was prepared as follows:

Anhydrous α,ω-diallyl poly(ethylene oxide) (PEODIAL) which had a molar mass of 520 g/mol and which was prepared by adapting the procedure disclosed in the publication Mei-Hui, Yang, Laing-Jong, Li, and Tsang-Feng, Ho, Synthesis and Characterization of polymethylsiloxane/poly(ethylene glycol)monomethyl ether copolymers, J. Ch. Colloid & Interface Soc. 3(17), 1994, 19-28 and α,ω-bis(dimethyl silyl hydride) poly(dimethyl siloxane) (PDMSDIH, Mₙ = 6000 g/mol) were weighed into a three-necked flask. The mass of the PEODIAL was 1.38 g (Mₙ = 520 g/mol, 5.28 mmol of allyl groups) and the mass of PDMSDIH was 12 g (4.8 mmol of hydride groups), the amount of allyl groups being 10 % greater than that of hydride groups. Thus an α,ω-diallyl-end-blocked final product was ensured.

In addition, toluene was weighed into the reaction vessel in an amount of 45 % by weight (7.2 g). The reaction mixture was stirred over a magnetic stirring plate at 200 rpm, and dry oxygen was bubbled through the mixture in order to prevent the deactivation of the catalyst. The temperature of the reaction mixture was raised to 60 °C. Thereafter the catalyst (Pt(0) divinyl tetramethyl disiloxane complex) was added to the reaction solution through the septum, cautiously one drop at the time. The amount of platinum was 50 ppm, calculated from the reactants. The polymerization was allowed to proceed for approximately 6 h, whereafter the completion of the polymerization was confirmed by IR (loss of the Si-H peak at 2130 cm⁻¹). For the removal of the toluene by distillation, the temperature was raised to 65 °C and the pressure was lowered to 5 mbar for a period of 30 min.

The amounts of the ingredients of the composition example were as follows:
- APEO-(-PMDS-APEO)ₙ 94.68 % by weight
- Platinum catalyst 0.5 % by weight
- Crosslinking agent 4.7 % by weight
- Inhibitor 0.12 % by weight

3.0 grams of the APEO-(-PMDS-APEO)ₙ, 0.0158 grams of the catalyst, 0.0038 g of the inhibitor, and 0.1489 g of the crosslinking agent were mixed together. The air bubbles were removed from the mixture, and the mixture was crosslinked in a hot-press at 110 °C for 15 minutes and was cured at 110 °C for 15 minutes.

### EXAMPLE 9

### Elastomer membrane prepared from composition type I

Ingredients used for the elastomer membrane:
- PEO-(PDMS-PEO)ₙ, where the amount of PEO was 5.0 % by weight and the vinyl content was 0.04 mmol/g.
- The DMS-VMS copolymer containing a silica filler was the same as in Example 2.
- Dichlorobenzoyl peroxide (Perkadox PD50 S, Nusil).

The PEO-(PDMS-PEO)ₙ used was prepared as follows:

0.528 g of anhydrous α,ω-divinyl ether poly(ethylene oxide) (PEODIVI) having a molar mass of 240 g/mol was weighed into a three-necked flask. 10 g of α,ω-bis(dimethyl silyl hydride)poly(dimethyl silyl siloxane) (PDMSDIH) having a molar mass of 6000 g/mol was weighed into the same vessel. The PDMSDIH contained hydride groups in an amount of 0.04 % by weight, and thus the amount of hydride groups in 10 grams was 4 mmol and the amount of PEODIVI vinyl groups was 4.4 mmol. Since the vinyl groups were present in excess (10 %) in the reaction, vinyl groups were obtained at both ends of the final product, a fact essential for the subsequent crosslinking. In addition, to facilitate mixing and to prevent the reaction from occurring too vigorously, toluene dried by distillation was added to the reaction mixture so that the proportion of toluene was 30 % by weight (4.5 g). The reaction solution was stirred over a magnetic stirring plate at 200 rpm, and dry oxygen was directed through the solution; this prevented the catalyst from converting to metallic form and thus prevented the deactivation of the catalyst. The reaction solution was heated to 50 °C, whereafter the catalyst (Pt(0) divinyl tetramethyl disiloxane complex) was added to the mixture through the septum. The amount of platinum was 50 ppm, calculated from the amount of the reactants. The catalyst was added dropwise, whereby hot spots in the reactor were avoided. After the adding of the catalyst the reaction was allowed to proceed for 2 h. Thereafter the completion of the reaction was confirmed by IR (loss of the Si-H peak at 2130 cm⁻¹). After the polymerization the reaction mixture was heated to 65 °C and the toluene was removed by vacuum distillation (5 mbar) in the course of 30 minutes.

The amounts of ingredients in the composition example were as follows:
- PEO-(PDMS-PEO)ₙ, 4.9 % by weight
- silica-filled DMS-VMS copolymer, 93.9 % by weight
- dichlorobenzoyl peroxide (Perkadox PD50 S, Nusil), 1.2 % by weight.

0.5 g of PEO-(PDMS-PEO)ₙ and 9.5 g of a DMS-VMS copolymer containing a filler were mixed together. 0.12 g of the peroxide catalyst was mixed with the homogeneous blend, and the blend was hardened at a temperature of +115 °C and a pressure of 200 bar for 5 minutes and was cured at +150 °C for 2 hours.

### EXAMPLE 10

### Elastomer membrane prepared from composition type J

Ingredients used for the preparation of the elastomer:
- PDMS-PEO graft copolymer the same as in Example 6
- Dichlorobenzoyl peroxide Perkadox PD50 S, Nusil

The amounts of the ingredients in the composition example were as follows:
- PDMS-PEO graft copolymer 98.8 % by weight
- Dichlorobenzoyl peroxide Perkadox PD50 S 1.2 % by weight

10 grams of the PDMS-PEO graft copolymer and 0.12 grams of Perkadox PD50 S were mixed together. The blend was hardened at a temperature of +115 °C and a pressure of 200 bar for 5 minutes and was cured at +150 °C for 2 hours.

### Permeation tests

Various compositions, in which the amount of PEO groups varied, were prepared of the above-mentioned composition types A - J. Composition types A - G were tested for the permeation rates of various drugs.

The assay apparatus described in the publication Yie W. Chien, Transdermal Controlled Systemic Medications, Marcel Dekker Inc., New York and Basel 1987, page 173, was used in the tests.

The drug fluxes (permeations) through membranes were measured with a two-compartment diffusion cell at 37 °C (side-by-side diffusion cell, Crown Glass Company). The apparatus consisted of two concentric cells (donor and receptor compartments) that were separated by the elastomer membrane to be investigated. The donor and receptor compartments were both jacketed and thermostated by an external circulating bath and each compartment had a magnetic stirrer. A drug solution and solvent (without drug) was added into the donor and the receptor compartments. At each predetermined time interval, samples were withdrawn from the receptor compartment and replaced with the same volume of solvent. The amount of the drug that permeated through the membrane was measured by HPLC. In all measurements, the thickness (0.4 mm) of the membrane and the surface area of the membranes were constant.

In the tests described below, the permeation rates of two different drugs through a 0.4-mm-thick elastomer membrane were measured by using the assay apparatus described above. The tables below show the effect of the concentration of PEO groups (% by weight of the said compositions) on the permeation rates of the different drugs for elastomers prepared from different composition types. The tables show the relative permeation as compared with a commercial crosslinked dimethyl siloxane-vinyl methyl siloxane elastomer (Mₙ approximately 400,000 g/mol) containing a silica filler.

| **Drug 1:** Levonorgestrel | | |
|---|---|---|
| Composition type | PEO concentration % by weight | Relative permeation |
| comparison | 0 | 1 |
| A | 28.0 | 14.5 |
| B | 3.8 | 1.5 |
| B | 4.1 | 2.0 |
| B | 5.0 | 2.3 |

| **Drug 2:** 17-β-Estradiol | | |
|---|---|---|
| Composition type | PEO concentration % by weight | Relative permeation |
| comparison | 0 | 1 |
| A | 11.6 | 21.3 |
| A | 26.4 | 110 |
| B | 7.8 | 13.3 |
| B | 9.8 | 24.4 |
| C | 3.4 | 4.6 |
| D | 52.3 | 90.4 |
| E | 11.4 | 7.7 |
| F | 1.3 | 2.4 |
| G | 0.5 | 1.4 |

The permeation tests performed showed that an increasing concentration of PEO in the membrane increased the permeation rate for each composition type and for each drug tested, regardless of whether the drug concerned was hydrophilic or lipophilic.

An elastomer composition according to the invention is, for example, highly suited for controlling, in implants and in intrauterine and intravaginal devices, the permeation rates of drugs having hormonal action.

The most important drugs having hormonal action include antiprogestins, progestins, estradiols and androgens.

The above embodiments of the invention are only examples of the implementation of the idea of the invention. For a person skilled in the art it is clear that the different embodiments of the invention may vary within the framework of the claims presented below.

## Claims

1. A membrane or matrix for controlling the permeation rate of a drug, said membrane or matrix comprising a siloxane-based elastomer composition comprising at least one elastomer and possibly a non-crosslinked polymer, **characterized in that** the elastomer composition comprises poly(alkylene oxide) groups, and that the poly(alkylene oxide) groups are present in the elastomer or polymer as alkoxy-terminated grafts of polysiloxane units, or as blocks, the said grafts or blocks being linked to the polysiloxane units by silicon-carbon bonds, or as a mixture of these forms.

2. The membrane or matrix according to Claim 1, **characterized in that** the elastomer composition is an elastomer made up of polysiloxane units which comprise poly(alkylene oxide) groups.

3. The membrane or matrix according to Claim 1 or 2, **characterized in that** the poly(alkylene oxide) groups are poly(ethylene oxide) groups (PEO groups).

4. The membrane or matrix according to Claim 2 or 3, **characterized in that** the formula of the polysiloxane groups is
-(SiR'R"O)_{q}SiR'R"-
where R' and R" are
- partly free groups, which are the same or different and which are a lower alkyl group, or a phenyl group, in which case the said alkyl or phenyl group may be substituted or unsubstituted, or alkoxy-terminated poly(alkylene oxide) groups having the formula 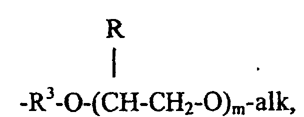 where alk is a lower alkyl group, suitably methyl, R is hydrogen or a lower alkyl, R³ is a straight-chain or branched C₂ - C₆ alkyl, and m is 1...30,
- partly bonds formed from the hydrogen or alkylene groups to other polymer chains in the elastomer, and
- possibly partly unreacted groups, such as hydrogen, vinyl or vinyl-terminated alkene, and
- q is 1...3000.

5. The membrane or matrix according to Claim 4, **characterized in that** the free R' and R" groups are a lower alkyl group, preferably methyl.

6. The membrane or matrix according to Claim 2 or 3, **characterized in that** the poly(alkylene oxide) groups are present in the elastomer in the form of poly(alkylene oxide) blocks having the formula 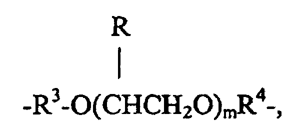 or 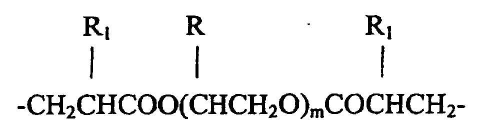 where R is hydrogen, a lower alkyl or phenyl, R₁ is hydrogen or a lower alkyl, R³ and R⁴ are the same or different and are straight-chain or branched C₂ - C₆ alkyl groups, and m is 1...30.

7. The membrane or matrix according to Claim 1, **characterized in that** the elastomer composition is made up of two elastomers interlaced one inside the other, in which case
- the first elastomer comprises poly(alkylene oxide) groups, and that the poly(alkylene oxide) groups are present in the said elastomer as alkoxy-terminated grafts of polysiloxane units, or as blocks, in which case the said grafts or blocks are linked to the polysiloxane units by silicon-carbon bonds, or as a mixture of these forms, and that
- the second elastomer is a siloxane-based elastomer.

8. The membrane or matrix according to Claim 7, **characterized in that** the second elastomer is a poly(dimethyl siloxane)-based elastomer which possibly comprises poly(alkylene oxide) groups.

9. The membrane or matrix according to Claim 8, **characterized in that** the possible poly(alkylene oxide) groups of the second poly(dimethyl siloxane)-based elastomer are present in the form of alkoxy-terminated grafts of poly(dimethyl siloxane) units or as blocks, the said grafts or blocks being linked to the poly(dimethyl siloxane) units by silicon-carbon bonds, or as a mixture of these forms.

10. The membrane or matrix according to Claim 1, **characterized in that** the elastomer composition is a blend which comprises
- a siloxane-based elastomer and
- a straight-chain polysiloxane copolymer which comprises poly(alkylene oxide) groups, in which case the poly(alkylene oxide) groups are present in the said polymer as alkoxy-terminated grafts of polysiloxane units, or as blocks, the said grafts or blocks being linked to the polysiloxane units by silicon-carbon bonds, or a mixture of these forms.

11. The membrane or matrix according to Claim 10, **characterized in that** the poly(alkylene oxide) groups are poly(ethylene oxide) groups (PEO groups).

12. The membrane or matrix according to Claim 10 or 11, **characterized in that** the formula of the polysiloxane groups is
-(SiR'R"O)_{q}SiR'R"-
where R' and R" are the same or different and are a lower alkyl group, or a phenyl group, in which case the said alkyl or phenyl group may be substituted or unsubstituted, or alkoxy-terminated poly(alkylene oxide) groups having the formula 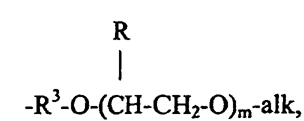 where alk is a lower alkyl group, suitably methyl, R is hydrogen or a lower alkyl, R³ is a straight or branched C₂ - C₆ alkyl group, m is 1...30, and q is 1...3000.

13. The membrane or matrix according to Claim 12, **characterized in that** the free R' and R" groups are lower alkyl groups, preferably methyl.

14. The membrane or matrix according to Claim 10 or 11, **characterized in that** the poly(alkylene oxide) groups are present in the straight-chain polysiloxane polymer in the form of poly(alkylene oxide) blocks having the formula 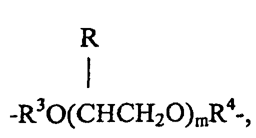 or 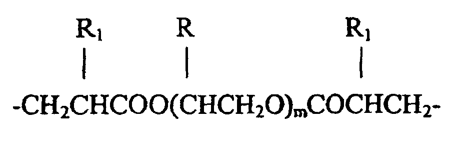 where R is hydrogen, a lower alkyl or phenyl, R₁ is hydrogen or a lower alkyl, R³ and R⁴ are the same or different and are straight-chain or branched C₂ - C₆ alkyl groups, and m is 1...30.

15. The membrane or matrix according to Claim 10, **characterized in that** the siloxane-based elastomer is made up of poly(dimethyl siloxane).

16. The membrane or matrix according to any of Claims 10 - 15, **characterized in that** the siloxane-based elastomer comprises poly(alkylene oxide) groups, and that the poly(alkylene oxide) groups are present in the elastomer or polymer as alkoxy-terminated grafts of polysiloxane units, or as blocks, the said grafts or blocks being linked to the polysiloxane units by silicon-carbon bonds, or as a mixture of these forms.

17. The membrane or matrix according to any of Claims 1 - 16, **characterized in that** it contains a filler, suitably silica.

18. A method for the preparation of a siloxane-based elastomer which comprises poly(alkylene oxide) groups and is intended for use in a membrane or matrix controlling the permeation rate of drugs, **characterized in that**
a) a vinyl-functional polymer component and a hydride-functional component are crosslinked in the presence of a catalyst, or
b) a polymer component is crosslinked in the presence of a peroxide catalyst, and that
I) the vinyl-functional polymer component is
a) a vinyl-functional polysiloxane having the formula
R'-Sir'R"O(SiR'R"O)ᵣSiR'R"R'
where R' and R" are the same or different and are a lower alkyl group or a phenyl group, in which case the said alkyl or phenyl group may be substituted or unsubstituted, and where some of the substituents R' and/or R" have been substituted for by vinyl groups, and r is 1...27000, or
b) an alkenyl terminated polysiloxane-based block copolymer having the formula
T(AB)ₓAT (I),
where
A = -(SiR'R"O)_{q}SiR'R"-, where R' and R" are the same or different and are a lower alkyl group or a phenyl group, in which case the said alkyl or phenyl group may be substituted or unsubstituted;
B is a poly(alkylene oxide) having the formula 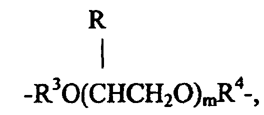 or 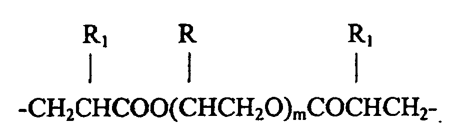 and T is 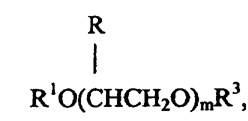 or 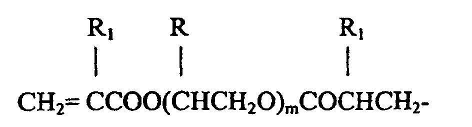 where R is hydrogen, a lower alkyl or phenyl, R₁ is hydrogen or a lower alkyl, R³ and R⁴ are the same or different and are straight-chain or branched C₂ - C₆ alkylene groups, R¹ is a straight-chain or branched C₂ - C₆ alkenyl group, m is 1...30, q is 1...3000, and x is 0...100, or
c) a vinyl-functional polysiloxane copolymer having the formula
R'-SiR'R"O(SiR'R"O)ᵣ(SiR'R"O)ₚSiR'R"-R'
- where, in the first block, R' and R" are the same or different and are a lower alkyl group, or a phenyl group, in which case the said alkyl or phenyl group may be substituted or unsubstituted, and where some of the substituents R' and/or R" have been substituted for by vinyl groups, and r is 1...27000, and
- where, in the second block, R' is a lower alkyl group, or an alkoxy-terminated poly(alkylene oxide) group having the formula 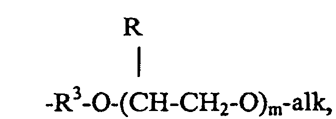 where alk is a lower alkyl group, suitably methyl, R³ is a straight or branched C₂ - C₆ alkyl group, R is hydrogen or a lower alkyl group, and m is 1...30, or R' is a phenyl group, in which case the said alkyl or phenyl group may be substituted or unsubstituted, and R" is a lower alkyl group or a phenyl group, in which case the said alkyl or phenyl group may be substituted or unsubstituted, and p is 1...5000, or
d) α,ω-dialkenyl poly(alkylene oxide) having the formula 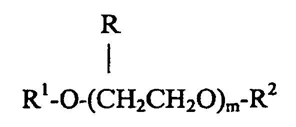 where R is hydrogen or a lower alkyl, R¹ and R² are the same or different straight-chain or branched C₂ - C₆ alkenyl groups, and m is 1...30, or
e) a blend of at least two of the above-mentioned components a) - d), and that
II) the hydride-functional component is
a) a hydride-functional siloxane which may be straight-chain, star shaped, branched or cyclic, or
b) a hydride-terminated siloxane-based block copolymer having the formula
T(BA)ₓBT (II),
where
T = H-SiR'R"O(SiR'R"O)_{q}SiR'R"-,
A = -SiR'R"O(SiR'R"O)_{q}SiR'R"-, where R' and R" are the same or different and are a lower alkyl group or a phenyl group, in which case the said alkyl or phenyl group may be substituted or unsubstituted;
B is a poly(alkylene oxide) having the formula 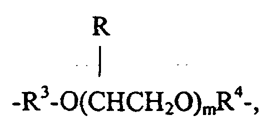 or 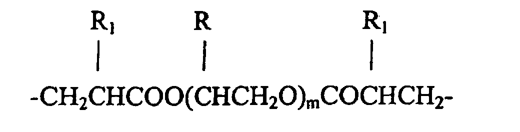 where R is hydrogen, a lower alkyl or phenyl, R₁ is hydrogen or a lower alkyl, R³ and R⁴ are the same or different and are straight-chain or branched C₂ - C₆ alkyl groups, m is 1...30, q is 1...3000, and x is 0...100, or
c) a blend of the above-mentioned components a) and b).

19. The method according to Claim 18, **characterized in that** the amounts of the vinyl-functional component and the hydride-functional component are selected so that the ratio of the molar amount of hydrides to the molar amount of double bonds is at minimum 1.

20. The method according to Claim 18, **characterized in that** the hydride-functional siloxane copolymer is straight-chain, and that its formula is
R'-SiR'R"O(SiR'R"O)ᵣSiR'R"R'
where R' and R" are the same or different and are a lower alkyl group or a phenyl group, in which case the said alkyl or phenyl group may be substituted or unsubstituted, and where some of the substituents R' and/or R" have been substituted for by hydrogen, and r is 1...27000.

21. The method according to any of Claims 18 - 20, **characterized in that** the vinyl-functional polymer component contains a filler, suitably silica.

## Patentansprüche

1. Membran oder Matrix zur Steuerung der Permeationsrate eines Wirkstoffs, wobei die Membran oder Matrix eine Elastomerzusammensetzung auf Siloxanbasis umfasst, die mindestens ein Elastomer und möglicherweise ein nicht-vernetztes Polymer umfasst, **dadurch gekennzeichnet, dass** die Elastomerzusammensetzung Poly(alkylenoxid)-Gruppen umfasst und dass die Poly(alkylenoxid)-Gruppen in dem Elastomer oder Polymer als Alkoxy-terminierte Aufpfropfungen der Polysiloxaneinheiten oder als Blöcke, wobei die Aufpfropfungen oder Blöcke durch Silicium-Kohlenstoff-Bindungen an die Polysiloxaneinheiten gebunden sind, oder als ein Gemisch dieser Formen vorliegen.

2. Membran oder Matrix nach Anspruch 1, **dadurch gekennzeichnet, dass** die Elastomerzusammensetzung ein Elastomer ist, das aus Polysiloxaneinheiten besteht, die Poly(alkylenoxid)-Gruppen umfassen.

3. Membran oder Matrix nach Anspruch 1 oder 2, **dadurch gekennzeichnet, dass** die Poly(alkylenoxid)-Gruppen Poly(ethylenoxid)-Gruppen (PEO-Gruppen) sind.

4. Membran oder Matrix nach Anspruch 2 oder 3, **dadurch gekennzeichnet, dass** die Formel der Polysiloxangruppen
-(SiR'R''O)_{q}SiR'R''-
lautet, wobei R' und R"
- teilweise freie Gruppen, die gleich oder verschieden sind und die eine Niederalkylgruppe oder eine Phenylgruppe, wobei die Alkyl- oder Phenylgruppe substituiert oder unsubstituiert sein kann, oder Alkoxy-terminierte Poly(alkylenoxid)-Gruppen der Formel 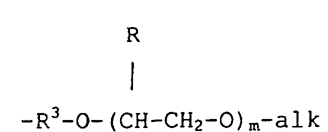 sind, worin alk eine Niederalkylgruppe, geeigneterweise Methyl ist, R Wasserstoff oder eine Niederalkylgruppe ist, R³ ein geradkettiges oder verzweigtes C₂-C₆-Alkyl ist und m 1...30 ist,
- teilweise Bindungen, die von Wasserstoff oder Alkylengruppen zu anderen Polymerketten in dem Elastomer gebildet werden, und
- möglicherweise teilweise nicht umgesetzte Gruppen, wie Wasserstoff, Vinyl oder Vinyl-terminiertes Alken, sind und
- q 1...3000 ist.

5. Membran oder Matrix nach Anspruch 4, **dadurch gekennzeichnet, dass** die freien R' - und R'' -Gruppen eine Niederalkylgruppe, vorzugsweise Methyl, sind.

6. Membran oder Matrix nach Anspruch 2 oder 3, **dadurch gekennzeichnet, dass** die Poly(alkylenoxid)-Gruppen in dem Elastomer in der Form von Poly(alkylenoxid)-Blöcken der Formel 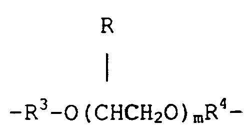 oder 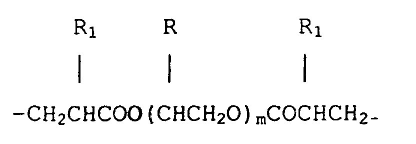 vorliegen, worin R Wasserstoff, Niederalkyl oder Phenyl ist, R₁ Wasserstoff oder Niederalkyl ist, R³ und R⁴ dasselbe oder verschieden sind und für geradkettige oder verzweigte C₂-C₆-Alkylgruppen stehen und m 1...30 ist.

7. Membran oder Matrix nach Anspruch 1, **dadurch gekennzeichnet, dass** die Elastomerzusammensetzung aus zwei Elastomeren besteht, die ineinander verflochten sind, wobei
- das erste Elastomer Poly(alkylenoxid)-Gruppen umfasst und die Poly(alkylenoxid)-Gruppen in dem Elastomer als Alkoxy-terminierte Aufpfropfungen der Polysiloxaneinheiten oder als Blöcke, wobei die Aufpfropfungen oder Blöcke durch Silicium-Kohlenstoff-Bindungen an die Polysiloxaneinheiten gebunden sind, oder als ein Gemisch dieser Formen vorliegen und
- das zweite Elastomer ein Elastomer auf Siloxanbasis ist.

8. Membran oder Matrix nach Anspruch 7, **dadurch gekennzeichnet, dass** das zweite Elastomer ein Elastomer auf Poly(dimethylsiloxan)-Basis ist, das möglicherweise Poly(alkylenoxid)-Gruppen umfasst.

9. Membran oder Matrix nach Anspruch 8, **dadurch gekennzeichnet, dass** die möglichen Poly(alkylenoxid)-Gruppen des zweiten Elastomers auf Poly(dimethylsiloxan)-Basis in der Form von Alkoxy-terminierten Aufpfropfungen der Poly(dimethylsiloxan)-Einheiten oder als Blöcke, wobei die Aufpfropfungen oder Blöcke durch Silicium-Kohlenstoff-Bindungen an die Poly(dimethylsiloxan)-Einheiten gebunden sind, oder als ein Gemisch dieser Formen vorliegen.

10. Membran oder Matrix nach Anspruch 1, **dadurch gekennzeichnet, dass** die Elastomerzusammensetzung ein Gemisch ist, das
- ein Elastomer auf Siloxanbasis und
- ein geradkettiges Polysiloxan-Copolymer umfasst, das Poly(alkylenoxid)-Gruppen umfasst, wobei die Poly(alkylenoxid)-Gruppen in dem Polymer als Alkoxy-terminierte Aufpfropfungen der Polysiloxaneinheiten oder als Blöcke, wobei die Aufpfropfungen oder Blöcke durch Silicium-Kohlenstoff-Bindungen an die Polysiloxaneinheiten gebunden sind, oder ein Gemisch dieser Formen vorliegen.

11. Membran oder Matrix nach Anspruch 10, **dadurch gekennzeichnet, dass** die Poly(alkylenoxid)-Gruppen Poly(ethylenoxid)-Gruppen (PEO-Gruppen) sind.

12. Membran oder Matrix nach Anspruch 10 oder 11, **dadurch gekennzeichnet, dass** die Formel der Polysiloxangruppen
-(SiR'R''O)_{q}SiR'R''-
lautet, worin R' und R" dasselbe oder verschieden sind und eine Niederalkylgruppe oder eine Phenylgruppe, wobei die Alkyl- oder Phenylgruppe substituiert oder unsubstituiert sein kann, oder Alkoxy-terminierte Poly(alkylenoxid)-Gruppen der Formel 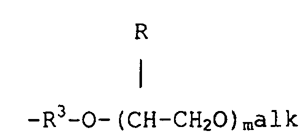 sind, worin alk eine Niederalkylgruppe, geeigneterweise Methyl ist, R Wasserstoff oder Niederalkyl ist, R³ eine geradkettige oder verzweigte C₂-C₆-Alkylgruppe ist, m 1...30 ist und q 1...3000 ist.

13. Membran oder Matrix nach Anspruch 12, **dadurch gekennzeichnet, dass** die freien R' - und R'' -Gruppen Niederalkylgruppen, vorzugsweise Methyl, sind.

14. Membran oder Matrix nach Anspruch 10 oder 11, **dadurch gekennzeichnet, dass** die Poly(alkylenoxid)-Gruppen in dem geradkettigen Polysiloxan-Polymer in der Form von Poly(alkylenoxid)-Blöcken der Formel 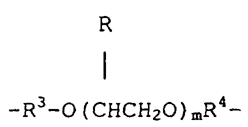 oder 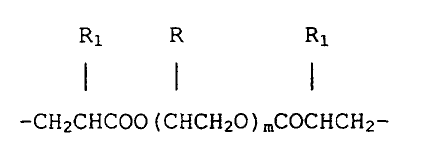 vorliegen, worin R Wasserstoff, Niederalkyl oder Phenyl ist, R₁ Wasserstoff oder Niederalkyl ist, R³ und R⁴ dasselbe oder verschieden sind und geradkettige oder verzweigte C₂-C₆-Alkylgruppen sind und m 1...30 ist.

15. Membran oder Matrix nach Anspruch 10, **dadurch gekennzeichnet, dass** das Elastomer auf Siloxanbasis aus Poly(dimethylsiloxan) besteht.

16. Membran oder Matrix nach einem der Ansprüche 10 bis 15, **dadurch gekennzeichnet, dass** das Elastomer auf Siloxanbasis Poly(alkylenoxid)-Gruppen umfasst und dass die Poly(alkylenoxid)-Gruppen in dem Elastomer oder Polymer als Alkoxy-terminierte Aufpfropfungen der Polysiloxaneinheiten oder als Blöcke, wobei die Aufpfropfungen oder Blöcke durch Silicium-Kohlenstoff-Bindungen an die Polysiloxaneinheiten gebunden sind, oder als ein Gemisch dieser Formen vorliegen.

17. Membran oder Matrix nach einem der Ansprüche 1 bis 16, **dadurch gekennzeichnet, dass** sie einen Füllstoff, geeigneterweise Siliciumdioxid, enthält.

18. Verfahren zur Herstellung eines Elastomers auf Siloxanbasis, das Poly(alkylenoxid)-Gruppen umfasst und zur Verwendung in einer Membran oder Matrix zur Steuerung der Permeationsrate von Wirkstoffen bestimmt ist, **dadurch gekennzeichnet, dass**
a) eine Vinyl-funktionelle Polymerkomponente und eine Hydrid-funktionelle Komponente in Gegenwart eines Katalysators vernetzt werden oder
b) eine Polymerkomponente in Gegenwart eines Peroxidkatalysators vernetzt wird und das
I) die Vinyl-funktionelle Polymerkomponente
a) ein Vinyl-funktionelles Polysiloxan der Formel
R'-SiR'R''O(SiR'R''O)ᵣSiR'R''R'
ist, worin R' und R'' dasselbe oder verschieden sind und für eine Niederalkylgruppe oder eine Phenylgruppe stehen, wobei die Alkyl- oder Phenylgruppe substituiert oder unsubstituiert sein kann und worin einige der Substituenten R' und/oder R" durch Vinylgruppen substituiert sind und r 1...27000 ist, oder
b) ein Alkenyl-terminiertes Blockcopolymer auf Polysiloxanbasis der Formel
T(AB)ₓAT (I),
ist, worin
A = -(SiR'R''O)_{q}SiR'R''-, worin R' und R'' dasselbe oder verschieden sind und für eine Niederalkylgruppe oder eine Phenylgruppe stehen, wobei die Alkyl- oder Phenylgruppe substituiert oder unsubstituiert sein kann;
B ein Poly(alkylenoxid) der Formel 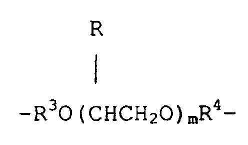 oder 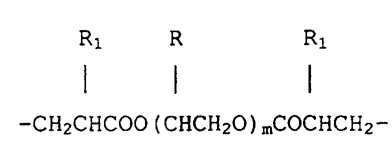 ist und T 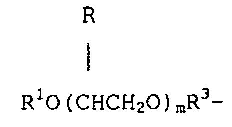 oder 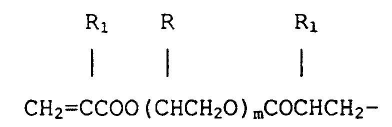 ist,
worin R Wasserstoff, Niederalkyl oder Phenyl ist, R₁ Wasserstoff oder Niederalkyl ist, R³ und R⁴ dasselbe oder verschieden sind und geradkettige oder verzweigte C₂-C₆-Alkylengruppen sind, R¹ eine geradkettige oder verzweigte C₂-C₆-Alkenylgruppe ist, m 1...30 ist, q 1...3000 ist und x 0...100 ist, oder
c) ein Vinyl-funktionelles Polysiloxan-Copolymer der Formel
R'-SiR'R''O(SiR'R''O)ᵣ(SiR'R''O)ₚSiR'R''-R'
- worin im ersten Block R' und R" dasselbe oder verschieden sind und für eine Niederalkylgruppe oder eine Phenylgruppe stehen, wobei die Alkyl- oder Phenylgruppe substituiert oder unsubstituiert sein kann, und worin einige der Substituenten R' und/oder R'' durch Vinylgruppen substituiert sind und r 1...27000 ist, und
- worin im zweiten Block R' eine Niederalkylgruppe oder eine Alkoxy-terminierte Poly(alkylenoxid)-Gruppe der Formel 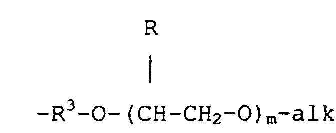 ist, worin alk eine Niederalkylgruppe, geeigneterweise Methyl, ist, R³ eine geradkettige oder verzweigte C₂-C₆-Alkylgruppe ist, R Wasserstoff oder eine Niederalkylgruppe ist und m 1...30 ist, oder R' eine Phenylgruppe ist, wobei die Alkyl- oder Phenylgruppe substituiert oder unsubstituiert sein kann, und R'' eine Niederalkylgruppe oder eine Phenylgruppe ist, wobei die Alkyl- oder Phenylgruppe substituiert oder unsubstituiert sein kann, und p 1...5000 ist, oder
d) ein α,ω-Dialkylenpoly(alkylenoxid) der Formel 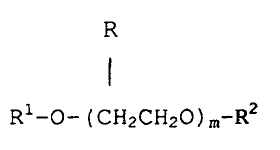 ist, worin R Wasserstoff oder Niederalkyl ist, R¹ und R² gleiche oder verschieden geradkettige oder verzweigte C₂-C₆-Alkenylgruppen sind und m 1...30 ist, oder
e) ein Gemisch aus mindestens 2 der voranstehend aufgeführten Komponenten a)-d) ist und dass
II) die Hydrid-funktionelle Komponente
a) ein Hydrid-funktionelles Siloxan ist, das geradkettig, sternförmig, verzweigt oder cyclisch sein kann, oder
b) ein Hydrid-terminiertes Blockcopolymer auf Siloxanbasis der Formel
T(BA)ₓBT (II)
ist, worin
T = H-SiR'R''O(SiR'R''O)_{q}SiR'R''-,
A = -SiR'R''O(SiR'R''O)_{q}SiR'R''-, worin R' und R" dasselbe oder verschieden sind und für eine Niederalkylgruppe oder eine Phenylgruppe stehen, wobei die Alkyl- oder Phenylgruppe substituiert oder unsubstituiert sein kann;
B ein Poly(alkylenoxid) der Formel 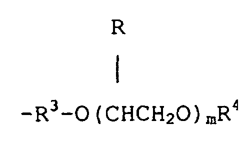 oder 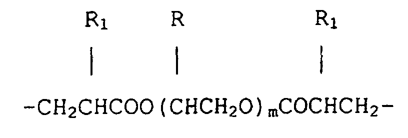 ist,
worin R Wasserstoff, Niederalkyl oder Phenyl ist, R₁ Wasserstoff oder Niederalkyl ist, R³ und R⁴ dasselbe oder verschieden sind und für geradkettige oder verzweigte C₂-C₆-Alkylgruppen stehen, m 1...30 ist, q 1...3000 ist und x 0...100 ist, oder
c) ein Gemisch der voranstehend aufgeführten Komponenten a) und b) ist.

19. Verfahren nach Anspruch 18, **dadurch gekennzeichnet, dass** die Mengen der Vinyl-funktionellen Komponente und der Hydrid-funktionellen Komponente so gewählt werden, dass das Verhältnis der molaren Menge an Hydriden zur molaren Menge an Doppelbindungen mindestens 1 beträgt.

20. Verfahren nach Anspruch 18, **dadurch gekennzeichnet, dass** das Hydrid-funktionelle Siloxancopolymer geradkettig ist und seine Formel
R'-SiR'R''O(SiR'R''O)ᵣSiR'R''R'
lautet, worin R' und R" dasselbe oder verschieden sind und für eine Niederalkylgruppe oder eine Phenylgruppe stehen, wobei die Alkyl- oder Phenylgruppe substituiert oder unsubstituiert sein kann, und worin einige der Substituenten R' und/oder R" durch Wasserstoff substituiert sind und r 1...27000 ist.

21. Verfahren nach einem der Ansprüche 18 bis 20, **dadurch gekennzeichnet, dass** die Vinyl-funktionelle Polymerkomponente einen Füllstoff, geeigneterweise Siliciumdioxid, enthält.

## Revendications

1. Membrane ou matrice pour réguler la vitesse de perméabilité d'un médicament, ladite membrane ou matrice comprenant une composition élastomère à base de siloxane comprenant au moins un élastomère et éventuellement un polymère non réticulé, **caractérisée en ce que** la composition élastomère comprend des groupes poly(oxyde d'alkylène) et **en ce que** les groupes poly(oxyde d'alkylène) sont présents dans l'élastomère ou le polymère sous forme de greffes à terminaison alcoxy de motifs polysiloxane ou sous forme de séquences, lesdites greffes ou séquences étant liées aux motifs polysiloxane par des liaisons silicium-carbone, ou sous forme d'un mélange de ces formes.

2. Membrane ou matrice selon la revendication 1, **caractérisée en ce que** la composition élastomère est un élastomère composé de motifs polysiloxane qui comprennent des groupes poly(oxyde d'alkylène).

3. Membrane ou matrice selon la revendication 1 ou 2, **caractérisée en ce que** les groupes poly(oxyde d'alkylène) sont des groupes poly(oxyde d'éthylène) (groupes PEO).

4. Membrane ou matrice selon la revendication 2 ou 3, **caractérisée en ce que** la formule des groupes polysiloxane est
-(SiR'R"O)_{q}SiR'R"-
dans laquelle R' et R" représentent
- en partie des groupes libres qui sont identiques ou différents et qui sont un groupe alkyle inférieur ou un groupe phényle, auquel cas ledit groupe alkyle ou phényle peut être substitué ou non substitué, ou des groupes poly(oxyde d'alkylène) à terminaison alcoxy de formule 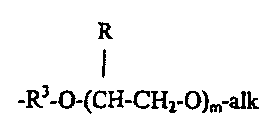 dans laquelle alk représente un groupe alkyle inférieur, de façon appropriée un groupe méthyle, R représente un atome d'hydrogène ou un groupe alkyle inférieur, R³ représente un groupe alkyle en C₂ à C₆ à chaîne droite ou ramifiée et m vaut 1 à 30,
- en partie des liaisons formées entre l'atome d'hydrogène ou les groupes alkylène et d'autres chaînes polymères de l'élastomère et
- éventuellement en partie des groupes n'ayant pas réagi tels qu'un atome d'hydrogène, un groupe vinyle ou alcène à terminaison vinyle, et
q vaut 1 à 3 000.

5. Membrane ou matrice selon la revendication 4, **caractérisée en ce que** les groupes libres R' et R" représentent un groupe alkyle inférieur, de préférence un groupe méthyle.

6. Membrane ou matrice selon la revendication 2 ou 3, **caractérisée en ce que** les groupes poly(oxyde d'alkylène) sont présents dans l'élastomère sous forme de séquences poly(oxyde d'alkylène) de formule 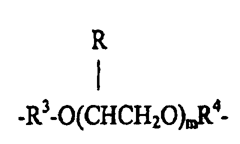 ou 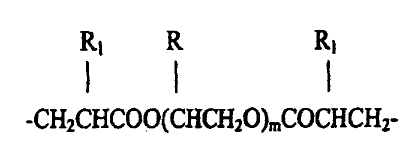 dans lesquelles R représente un atome d'hydrogène, un groupe alkyle inférieur ou phényle, R₁ représente un atome d'hydrogène ou un groupe alkyle inférieur, R³ et R⁴ sont identiques ou différents et représentent des groupes alkyle en C₂ à C₆ à chaîne droite ou ramifiée, et m vaut 1 à 30.

7. Membrane ou matrice selon la revendication 1, **caractérisée en ce que** la composition élastomère se compose de deux élastomères entrelacés l'un dans l'autre, auquel cas
- le premier élastomère comprend des groupes poly(oxyde d'alkylène) et **en ce que** les groupes poly(oxyde d'alkylène) sont présents dans ledit élastomère sous forme de greffes à terminaison alcoxy de motifs polysiloxane ou sous forme de séquences, auquel cas lesdites greffes ou séquences sont liées aux motifs polysiloxane par des liaisons silicium-carbone, ou sous forme d'un mélange de ces formes et **en ce que**
- le deuxième élastomère est un élastomère à base de siloxane.

8. Membrane ou matrice selon la revendication 7, **caractérisée en ce que** le deuxième élastomère est un élastomère à base de poly(diméthylsiloxane) qui comprend éventuellement des groupes poly(oxyde d'alkylène).

9. Membrane ou matrice selon la revendication 8, **caractérisée en ce que** les groupes poly(oxyde d'alkylène) éventuels du deuxième élastomère à base de poly(diméthylsiloxane) sont présents sous forme de greffes à terminaison alcoxy de motifs poly(diméthylsiloxane) ou sous forme de séquences, lesdites greffes ou séquences étant liées aux motifs poly(diméthylsiloxane) par des liaisons silicium-carbone, ou sous forme d'un mélange de ces formes.

10. Membrane ou matrice selon la revendication 1, **caractérisée en ce que** la composition élastomère est un mélange qui comprend
- un élastomère à base de siloxane, et
- un copolymère polysiloxane à chaîne droite qui comprend des groupes poly(oxyde d'alkylène), auquel cas les groupes poly(oxyde d'alkylène) sont présents dans ledit polymère sous forme de greffes à terminaison alcoxy de motifs polysiloxane ou sous forme de séquences, lesdites greffes ou séquences étant liées aux motifs polysiloxane par des liaisons silicium-carbone, ou sous forme d'un mélange de ces formes.

11. Membrane ou matrice selon la revendication 10, **caractérisée en ce que** les groupes poly(oxyde d'alkylène sont des groupes poly(oxyde d'éthylène) (groupes PEO).

12. Membrane ou matrice selon la revendication 10 ou 11, **caractérisée en ce que** la formule des groupes polysiloxane est
-(SiR'R"O)_{q}SiR'R"-
dans laquelle R' et R" sont identiques ou différents et représentent un groupe alkyle inférieur ou un groupe phényle, auquel cas ledit groupe alkyle ou phényle peut être substitué ou non substitué, ou des groupes poly(oxyde d'alkylène) à terminaison alcoxy de formule 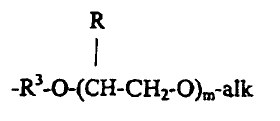 dans laquelle alk représente un groupe alkyle inférieur, de façon appropriée un groupe méthyle, R représente un atome d'hydrogène ou un groupe alkyle inférieur, R³ représente un groupe alkyle en C₂ à C₆ à chaîne droite ou ramifiée, m vaut 1 à 30 et q vaut 1 à 3 000.

13. Membrane ou matrice selon la revendication 12, **caractérisée en ce que** les groupes libres R' et R" représentent des groupes alkyle inférieur, de préférence des groupes méthyle.

14. Membrane ou matrice selon la revendication 10 ou 11, **caractérisée en ce que** les groupes poly(oxyde d'alkylène) sont présents dans le polymère polysiloxane à chaîne droite sous forme de séquences poly(oxyde d'alkylène) de formule 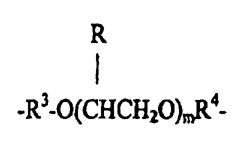 ou 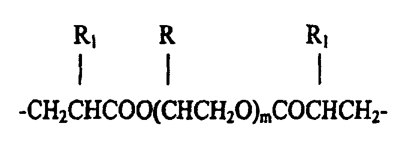 dans lesquelles R représente un atome d'hydrogène, un groupe alkyle inférieur ou phényle, R₁ représente un atome d'hydrogène ou un groupe alkyle inférieur, R³ et R⁴ sont identiques ou différents et représentent des groupes alkyles en C₂ à C₆ à chaîne droite ou ramifiée, et m vaut 1 à 30.

15. Membrane ou matrice selon la revendication 10, **caractérisée en ce que** l'élastomère à base de siloxane se compose de poly(diméthylsiloxane).

16. Membrane ou matrice selon l'une quelconque des revendications 10 à 15, **caractérisée en ce que** l'élastomère à base de siloxane comprend des groupes poly(oxyde d'alkylène), et **en ce que** les groupes poly(oxyde d'alkylène) sont présents dans l'élastomère ou le polymère sous forme de greffes à terminaison alcoxy de motifs polysiloxane ou sous forme de séquences, lesdites greffes ou séquences étant liées aux motifs polysiloxane par des liaisons silicium-carbone, ou sous forme d'un mélange de ces formes.

17. Membrane ou matrice selon l'une quelconque des revendications 1 à 16, **caractérisée en ce qu'**elle contient une charge, de façon appropriée de la silice.

18. Procédé de préparation d'un élastomère qui comprend des groupes poly(oxyde d'alkylène) et destiné à être utilisé dans une membrane ou une matrice régulant la vitesse de perméabilité de médicaments, **caractérisé en ce que**
a) un composant polymère à fonction vinyle et un composant à fonction hydryre sont réticulés en présence d'un catalyseur, ou
b) un composant polymère est réticulé en présence d'un catalyseur peroxyde et **en ce que**
I) le composant polymère à fonction vinyle est
a) un polysiloxane à fonction vinyle de formule
R'-SiR'R"O(SiR'R"O)ᵣSiR'R"R'
dans laquelle R' et R" sont identiques ou différents et représentent un groupe alkyle inférieur ou un groupe phényle, auquel cas ledit groupe alkyle ou phényle peut être substitué ou non substitué, et dans laquelle certains des substituants R' et/ou R" sont substitués par des groupes vinyle, et r vaut 1 à 27 000, ou
b) un copolymère séquencé à base de polysiloxane à terminaison alcényle de formule
T(AB)ₓAT (I),
dans laquelle
A représente -(SiR'R"O)_{q}SiR'R"-, où R' et R" sont identiques ou différents et représentent un groupe alkyle inférieur ou un groupe phényle, auquel cas ledit groupe alkyle ou phényle peut être substitué ou non substitué ;
B représente un poly(oxyde d'alkylène) de formule 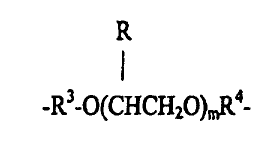 ou 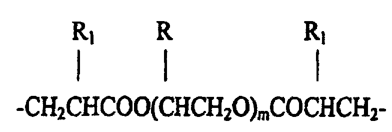 et T représente 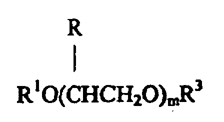 ou 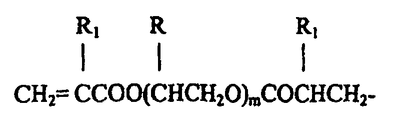 où R représente un atome d'hydrogène, un groupe alkyle inférieur ou phényle, R¹ représente un atome d'hydrogène ou un groupe alkyle inférieur, R³ et R⁴ sont identiques ou différents et représentent des groupes alkylène en C₂ à C₆ à chaîne droite ou ramifiée, R¹ représente un groupe alcényle en C₂ à C₆ à chaîne droite ou ramifiée, m vaut 1 à 30, q vaut 1 à 3 000 et x vaut 0 à 100, ou
c) un copolymère polysiloxane à fonction vinyle de formule
R'-SiR'R"O(SiR'R"O)ᵣ(SiR'R"O)ₚSiR'R"-R'
- dans laquelle, dans la première séquence, R' et R" sont identiques ou différents et représentent un groupe alkyle inférieur ou un groupe phényle, auquel cas ledit groupe alkyle ou phényle peut être substitué ou non substitué, et dans laquelle certains des substituants R' et/ou R" sont substitués par des groupes vinyles, et r vaut 1 à 27 000, et
- dans laquelle, dans la deuxième séquence, R' représente un groupe alkyle inférieur ou un groupe poly(oxyde d'alkylène) à terminaison alcoxy de formule 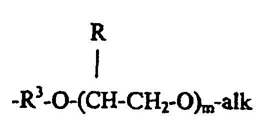 dans laquelle alk représente un groupe alkyle inférieur, de façon appropriée un groupe méthyle, R³ représente un groupe alkyle en C₂ à C₆ linéaire ou ramifié, R représente un atome d'hydrogène ou un groupe alkyle inférieur et m vaut 1 à 30, ou R' représente un groupe phényle, auquel cas ledit groupe alkyle ou phényle peut être substitué ou non substitué, et R" représente un groupe alkyle inférieur ou un groupe phényle, auquel cas ledit groupe alkyle ou phényle peut être substitué ou non substitué, et p vaut 1 à 5 000 ou
d) l'α,ω-dialcényl-poly(oxyde d'alkylène) de formule 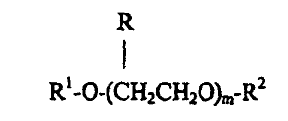 dans laquelle R représente un atome d'hydrogène ou un groupe alkyle inférieur, R¹ et R² représentent des groupes alcényle en C₂ à C₆ à chaîne droite ou ramifiée identiques ou différents, et m vaut 1 à 30, ou
e) un mélange d'au moins deux des composants a) à d) susmentionnés, et **en ce que**
II) le composant à fonction hybride est
a) un siloxane à fonction hydryre qui peut être à chaîne droite, en forme d'étoile, ramifié ou cyclique, ou
b) un copolymère séquencé à base de siloxane à terminaison hydryre de formule
T(BA)ₓBT (II),
dans laquelle
T représente H-SiR'R"O(SiR'R"O)_{q}SiR'R"-,
A représente -SiR'R"O(SiR'R"O)_{q}SiR'R"-, où R' et
R" sont identiques ou différents et représentent un groupe alkyle inférieur ou un groupe phényle, auquel cas ledit groupe alkyle ou phényle peut être substitué ou non substitué,
B représente un poly(oxyde d'alkylène) de formule 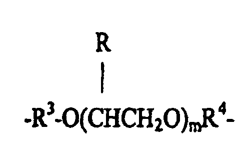 ou 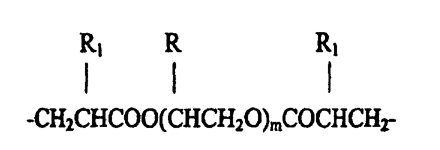 dans laquelle R représente un atome d'hydrogène, un groupe alkyle inférieur ou phényle, R₁ représente un atome d'hydrogène ou un groupe alkyle inférieur, R³ et R⁴ sont identiques ou différents et représentent des groupes alkyle en C₂ à C₆ à chaîne droite ou ramifiée, m vaut 1 à 30, q vaut 1 à 3 000 et x vaut 0 à 100, ou
c) un mélange des composants susmentionnés a) et b).

19. Procédé selon la revendication 18, **caractérisé en ce que** les quantités du composant à fonction vinyle et du composant à fonction hydryre sont choisies de sorte que le rapport de la quantité molaire des hydryres et de la quantité molaire des doubles liaisons vaut au moins 1.

20. Procédé selon la revendication 18, **caractérisé en ce que** le copolymère siloxane à fonction hydryre est une chaîne droite, et **en ce que** sa formule est
R'-SiR'R"O(SiR'R"O)ᵣSiR'R"R'
dans laquelle R' et R" sont identiques ou différents et représentent un groupe alkyle inférieur ou un groupe phényle, auquel cas ledit groupe alkyle ou phényle peut être substitué ou non substitué, et dans laquelle certains des substituants R' et/ou R" sont substitués par un atome d'hydrogène et r vaut 1 à 27 000.

21. Procédé selon l'une quelconque des revendications 18 à 20, **caractérisé en ce que** le composant polymère à fonction vinyle contient une charge, de façon appropriée de la silice.
